# EUROPEAN PATENT SPECIFICATION

(11) **EP 1 539 108 B1**
(45) Date of publication and mention of the grant of the patent: **20.02.2008**
(21) Application number: 03791488.4
(22) Date of filing: 25.08.2003
(51) Int. Cl.: A61K 9/127, A61K 31/573, A61K 31/57, A61K 31/58, A61P 35/00

(54) **VESICLE-ENCAPSULATED CORTICOSTEROIDS FOR THE TREATMENT OF CANCER**
VESIKEL-VERKAPSELTE KORTIKOSTEROIDE ZUR BEHANDLUNG VON KREBS
CORTICOSTEROIDES ENCAPSULES DANS UNE VESICULE POUR LE TRAITEMENT DU CANCER

(30) Priority: 27.08.2002 EP 02078525
(43) Date of publication of application: 15.06.2005
(73) Proprietor: Universiteit Utrecht, 3584 CS Utrecht (NL)
(72) Inventor: SCHIFFELERS, Raymond, Michel, NL-3023 JJ Rotterdam (NL); METSELAAR, Josbert, Maarten, NL-1094 ND Amsterdam (NL); MOLEMA, Grietje, NL-9718 BE Groningen (NL); STORM, Gerrit, NL-3813 CN Amersfoort (NL)
(74) Representative: Winckels, Johannes Hubertus F.
(86) International application number: PCT/NL2003/000596
(87) International publication number: WO 2004/019916

(56) References cited:
- EP-A- 1 044 679
- WO-A-02/45688
- US-A- 6 090 800
- METSELAAR J M ET AL: "LIPOSOMES FOR INTRAVENOUS DRUG TARGETING: DESIGN AND APPLICATIONS" MINI REVIEWS IN MEDICINAL CHEMISTRY, BENTHAM SCIENCE PUBLISHERS, HILVERSUM, NL, vol. 2, no. 4, August 2002 (2002-08), pages 319-329, XP001149516 ISSN: 1389-5575
- SHIBATA A ET AL: "PREPARATION AND CHARACTERIZATION OF LIPOSOMES INCORPORATING HYDROPHOBIC POLY(AMINO ACID)S WITH DIFFERENT SECONDARY STRUCTURE" CHEMICAL AND PHARMACEUTICAL BULLETIN, PHARMACEUTICAL SOCIETY OF JAPAN. TOKYO, JP, vol. 42, no. 5, 1 May 1994 (1994-05-01), pages 1151-1153, XP000466176 ISSN: 0009-2363
- GONZALEZ-ROTHI RICARDO J ET AL: "Pulmonary targeting of liposomal triamcinolone acetonide phosphate." PHARMACEUTICAL RESEARCH (NEW YORK), vol. 113, no. 11, 1996, pages 1699-1703, XP000998067 ISSN: 0724-8741

## Description

The present invention relates to the use of a composition comprising a corticosteroid in the treatment of cancer or the inhibition of cancer growth. More specifically, the invention relates to a method for targeting a corticosteroid to tumor tissue.

Even more specifically, the present invention aims to provide the use of such compositions in the treatment of non-lymphatic cancers, and preferably solid (non-hematological) malignancies and metastases (solid primary and secondary tumors), and is hence in the field of oncology.

Corticosteroids, such as glucocorticoids, have been proposed as active ingredients in the treatment of cancer. For example, Coleman has described in Biotherapy 4(1) (1992), 37-44 that in tumor therapy, glucocorticoids are often used for their anti-inflammatory and anti-emetic potential and for the treatment of haematological malignancies due to their efficient cytolytic activity on cells of lymphoid origin.

In the treatment of lymphatic cancer types, e.g. chronic and acute lymphatic leukaemia, Hodgkin and non-Hodgkin lymphomas, steroids have shown antitumor activity. In view of the fact that cells of lymphatic origin can be suppressed by corticosteroids (immuno suppressive activity) this is not surprising.

An example of such a treatment is disclosed in US-A-6,090,800 wherein vesicles, liposomes and micelles are described that contain lipid soluble steroid prodrugs. It is noted in this document that "steroids such as cortisone and dexamethasone are potent immune suppressants and are used to treat conditions such as auto-immune diseases, organ transplant rejection, arthritis, skin, mucosal membrane and ophthalmic inflammation, as well as neoplastic conditions such as lymphoma". In addition, this US patent teaches targetting to IL-2 receptors on T-cells.

Further reports in the 1980's and 1990's demonstrated that glucocorticoids could also decelerate solid tumor growth. Several studies showed that this effect was not directly aimed at the tumor cells, but rather mediated by interference with the tumor vascularization (see in this light, *e.g.*, Folkman et al. in Science 221 (4612) (1983), 719-723; Lee et al. in Cancer Res. 47(19) (1987), 5021-5024; McNatt et al. in J. Ocul. Pharmacol. Ther. 15(5) (1999) 413-423; and Crowley et al. in Oncology 45(4) (1988), 331-335). The exact mechanism of interference is, however, unclear. It has been suggested that it is mediated by inhibition of endothelial cell proliferation and migration (Cariou et al. in Cell. Biol. Int. Rep. 12(12) (1988), 1037-1047), basement membrane turnover (Folkman et al. (ibid); Ingber et al. in Endocrinology 119(4) (1986), 1768-1775), and/or inhibition of pro-angiogenic factors (like plaminogen activator and vascular endothelial growth factor) (Blei et al. in J. Cell Physiol. 155(3) (1993),568-578; Nauck et al. in Eur. J. Pharmacol. 341(2-3) (1998), 309-315). Furthermore, the hypothesis that inflammatory processes in and around the tumor are important in the angiogenic cascade suggests that glucocorticoids immunosuppressive action may also be involved (Bingle et al. in J. Pathol. 196(3) (2002), 254-265; O'Byrne & Dalgleish in Br. J. Cancer 85(4) (2001), 473-483).

One of the drawbacks of the strategy of using corticosteroids in solid tumor-therapy is the need for high dosing (typically 100-200 mg/kg per day) for prolonged periods of time to obtain significant tumor growth inhibition. The doses inevitably lead to severe side effects and have been shown to result in morbidity and mortality as a result of severe immune suppression in experimental animals.

It has been proposed in the state of the art that selective delivery of glucocorticoids to tumor tissue could be an attractive strategy to increase local drug concentrations. This would reduce the overall dose and, hence, decrease the likelihood of side effects. For instance, US-A-5,762,918 aimed at selective delivery of steroids to tumor endothelial cells via conjugation to polyanionics and especially to heparin (fragments). A maximal tumor inhibition of approximately 65% at a dose of cortisol of 35 mg/kg per day for 8 days is indicated.

More particularly, the corticosteroid-heparin conjugate described in said US-A-5,762,918 is claimed to be capable of delivering the corticosteroid more selectively to vascular endothelial cells. *In vivo* studies in mice demonstrated that a daily dose of 1 mg heparin-cortisol during 9 days resulted in a tumor reduction of 65 %, compared to 40 % reduction in mice treated with free cortisol and free heparin. The dosage per kg body weight was not specified but it has been found in both a mice model and a murine model that a cortisol-heparine conjugate is capable to obtain a 65 % reduction in tumor growth rate when administered at a daily dose of 35 mg/kg during 8 days.

A drawback of this approach is the chemical derivatization, required to bind the corticosteroid to the polyanionic, and particularly to heparin. The resultant binding between corticosteroid and heparin should be sufficiently stable to reach the tumor, but sufficiently reversible to allow dissociation of the corticosteroid. Furthermore, in order to overcome problems relating to the anticoagulant effect of heparin, specified heparins or its derivatives should be selected.

Although the Patentee mentioned on US-A-5,762,918 claims that the corticosteroid has an improved activity, because it is specifically delivered by the heparin to endothelial cells in and around the tumor, this mechanism is not demonstrated or supported by data. On the basis of the data presented, the only conclusion that can be drawn is that the heparin-corticosteroid conjugate has a higher activity. Moreover, it is noted that the pharmokinetic behaviour of the conjugate has not been studied. The heparin used has a low molecular weight, leading to efficient clearance by the kidneys. This makes that the conjugate probably also will be excreted by the kidneys, resulting in the fact that specific targeting of the conjugate to the tumor is not highly effective.

The conjugate is - as stated in US-A-5,762,918 - dependent on a stable coupling between corticosteroid and heparin and the cleavage of this coupling at the tumor site. The nature of the bond is critical and difficult to control. A premature cleavage or too less degradation of the coupling leads to less effective activity.

Said US-A-5,762,918 additionally states - while referring to the problems associated with too high doses of corticosteroids in the treatment of a.o. cancer - that
"another means of altering the pharmacologic properties of a drug, including altered biodistribution and reduced toxicity. Liposomal encapsulation involves the incorporation of molecules of the drug into a "capsule" of lipid materials, usually composed of uni- and multilamellar vesicles of various phospholipids. Unfortunately, the ability to form stable liposomes of a particular agent is somewhat limited, in that liposome stability is a function of a variety of parameters such as drug lipophilicity and other structural consideration. Thus, liposomal encapsulation has not proved to be applicable to a broad spectrum of agents. Furthermore, the ability of liposomal encapsulation to alter biodistribution is unpredictable at best. Thus, such encapsulation has not provided a particularly satisfactory broadly applicable means of targeting a selected drug in a uniform, tissue-specific manner."

There is an ongoing need for alternative therapies and therapeutical compositions.

In addition, it is an objective of the present invention to find a method to target corticosteroids to tumor tissue for or in the treatment, retardation or inhibition of cancer. Particularly, it is an objective to treat, retard or inhibit the growth of solid and non-lymphatic cancer types.

More specifically, the present invention is directed to a method to use microvesicles to encapsulate corticosteroids and use these systems for targeted tumor delivery.

In accordance with the present invention, it has now been found that a composition comprising a corticosteroid, encapsulated in particular types of microvesicles can be used to manufacture a medicament useful in the treatment of cancer.

Accordingly, the present invention relates to the use of a composition comprising a corticosteroid encapsulated in a long-circulating microvesicle for the manufacture of a medicament for the treatment of solid primary and/or secondary, tumors of a non-lymphatic cancer, and especially in oncology. The said composition was found to inhibit tumor growth, especially solid tumor growth, and can hence be used for this effect in the treatment of cancer, and especially in oncology.

The long-circulating microvesicles have very favorable pharmacokinetics, a favorable tissue distribution behavior and an efficient half life. Additionally, a stable association between corticosteroid and the carrier system, the microvesicles, is observed, while the loading with corticosteroid is efficient. Further a good biological availability at the site where activity is required is observed. Without wishing to be bound by any theory, it is hypothesized that the microvesicles have an interaction with macrophages in the tumor. This would mean that said cell type could be down regulated in tumor therapy, or that it may liberate the encapsulated corticosteroids to allow interaction with other cell types in and around the tumor.

In a preferred embodiment the long-circulating microvesicle is a liposome, a nanocapsule or a polymeric micelle. Preferably, the microvesicle has a neutral or negative charge out physiological conditions.

Other suitable long-circulating microvesicles can be based on lipoproteins, and especially high density lipoproteins and low density lipoproteins, and on lipoprotein mimetics or neo-lipoproteins.

It has been found that long-circulating microvesicles, and especially long-circulating liposomes, nanocapsules and polymeric micelles, are capable of efficiently delivering corticosteroids drugs to a specific site, i.e. to a tumor. In particular, the present invention provides a medicament for or in the treatment of cancer, suitable to administer corticosteroids, and especially glucocorticoids, in relatively low dosages. In accordance with the invention, effective inhibitions in tumor growth have been observed in particular embodiments with relatively low dosages of only 20 mg/kg body weight per week.

In addition, it has been found that by the use of corticosteroids present in the microvesicles of the invention, massive apoptosis of tumor cells in the center of the tumor is observed, which may be related to an impaired blood supply to the tumor. Further, there is an indication that the tumor is encapsulated by connective tissue. Particularly, in a mouse model, a loss of binding of the tumor to the underlying tissue is observed. Microscopic evaluation appeared to show formation of connective tissue encapsulating the tumor.

Without wishing to be bound by any theory, it is believed that microvesicles used in the present invention accumulate at sites of malignant tissues such as tumors as a result of the enhanced permeability of tumor vasculature as compared to healthy endothelium, allowing an improved localization and improved retention of the corticosteroid at these sites.

The long-circulating microvesicles used in accordance with the present invention typically have a mean particle diameter of less than 450, and preferably less than 300 nm as determined by Dynamic light scattering using a Malvern 4700^{™} system equipped with a He/Ne laser, and preferably of about 40 - 200 nm. Moreover, as can be seen in the working examples (*vide infra),* the microvesicles of the invention have a rather small polydispersity which means that the particle size distribution is narrow. Preferably, the polydispersity, which is calculated by the software belonging to the dynamic light scattering equipment, is less than 0.25, and more preferably less than 0.2.

Preferably, the long-circulating microvesicles used in the compositions of the invention are long-circulating liposomes. With such types of long-circulating microvesicles, it has been found *(vide infra)* that tumor growth can be reduced with more than 65 % and even up to 90% compared to controls at a dose of only 20 mg/kg body weight per week, within 2-3 weeks.

Such long-circulating liposomes are already known in the art, even in combination with corticosteroids, especially water soluble corticosteroids. More particularly, these known liposome systems are described to be useful in site-specific treatment of inflammatory disorders in WO-A-02/45688. For the preparation of suitable compositions to be used in the present invention, the preparation methods described in said WO-A-02/45688 are incorporated herein by reference.

In this document WO-A-02/45688, the liposome systems described in EP-A-0 662 820 are adapted to become "long-circulating".

EP-A-1 044 679 relates to liposomes having a drug included therein, which are said to have an ensured stability in blood. In addition these liposomes have an active targeting property to proteoglycan-rich areas. These areas are created because with some diseases, an over production of proteoglycans occurs; said proteoglycans keeping cell surfaces anionic. To target liposomes to these anionic surfaces, the liposomes need to be cationic in nature. Thereto, the said liposomes require the presence of a basic compound taking positive charge within a physiological pH range.

The liposomes of the present invention do not require the active targeting property described in EP-A-1 044 679. That is, no specific homing groups are required to selectively bring the microvesicles to the tumor sites. However, to increase the selectivity to an even higher extent, it is possible to attach or incorporate tumor specific antibodies or receptor ligands or food compounds at the outside surface of the microvesicles so as to increase the interaction possibilities with the tumor cells or the cells in or around the tumor.

The "targetting" of the neutral or optionally negatively charged liposomes of the present invention is ruled by the above-identified increased permeability in the tumor vasculature. That is, the present invention is for a major part based on passive accumulation, rather than active targetting.

The liposomes useful in the present invention should not have a positive charge and should hence not comprise components that give the liposomes a positive charge at physiological pH; that is at physiological pH, being a pH of between 6 and 8, the overall charge of the liposome to be used in the present invention should be neutral or negatively charged.

Preferred liposomes are based on non-charged vesicle-forming lipids. Neutral or non-charged vesicle-forming lipids lead to a suitable long circulation time. Typically, 5-10 mole% of negatively charged lipids may be present. Preferred lipids to be used to prepare the microvesicles used in the invention comprise saturated phospholipids and sphingolipids in combination with cholesterole and/or ergosterole and derivatives thereof.

To secure a suitable stability in the blood circulation system 10-50 mole% sterols should be present in the microvesicle material. Suitable liposome constituents are described in the above-identified WO-A-02/45688 and EP-A-0 662 820.

More preferably, the liposomes contain at least one type of polymer lipid conjugates, such as lipids derivatised with polyalkylene glycol, preferably with polyethylene glycol (PEG). Suitable polymer-lipid-conjugates have a molecular weight of between 200 and 30,000 Dalton.

Other suitable candidates to be used in these polymer-lipid-conjugates or water-soluble polymers such as: poly ((derivatized) carbohydrate)s, water-soluble vinylpolymers (*e.g*. poly(vinylpyrrolidone), polyacrylamide and poly(acryloylmorpholine) and poly(methyl/ethyl oxazone). These polymers are coupled to the lipid through conventional anchoring molecules.

Suitably, the concentration of polymer lipid conjugates is 0-20 mole%, and preferably 1-10 mole%, based upon the total molar ratio of the vesicle forming lipids.

The presence of these polymer-lipid-conjugates has a favorable effect on the circulation time. However, by carefully selecting specific lipid compositions an physical specifications suitable long circulation times can be obtained without using a polymer-lipid-conjugate. For example, 50-100 nm liposomes of distearylphopshatidylcholine and cholesterole and/or sphingolipids like sphingomyelin.

The liposomes may additionally contain one or more types of charged vesicle-forming lipids, *e.g*. phosphatidylglycerol, phosphatidylethanolamine, (di)stearylamine, phosphatidylserine, dioleoyl trimethylammonium propane, phosphatidic acids and cholesterol hemisuccinate.

Typically, the concentration of charged vesicle-forming lipids is 0-15 mole%, preferably 0-10 mole% based upon the molar ratio of the vesicle forming lipids.

Where in this description reference is made to charged/uncharged/amphiphatic, and so on, this reference relates to physiological conditions.

Hence, in a preferred embodiment the present invention relates to the use of a composition, wherein the microvesicle is a liposome comprising a non-charged vesicle-forming lipid, 0-20 mole% of an polymer-lipid conjugate and preferably a polyethyleneglycol, 0-50 mole% of a sterol, and 0-10 mol % of a charged vesicle-forming lipid. The liposomes have a preferred mean particle diameter in the size range between about 40 - 200 nm.

Polymeric micelles to be used in the present invention can be made in accordance with the method described in EP-A-1 072 617 adapted in accordance with the above-described method for the preparation of liposomes.

The long-circulating microvesicles have a circulation half life of at least 3 hours, and especially at least 6 hours. The circulation half life is, as the person skilled in the art appreciates, defined as the time at which the second linear phase of the logarithmic microvesicle, for instance liposomal, clearance profile reaches 50% of its initial concentration, which is the extrapolated plasma concentration at t=0.

In a preferred embodiment, the medicament to be used for or in the treatment of cancer is a medicament for parental or local application. Application through the oral or pulmonal route are however also possible.

Contrary to for instance the system taught in US-A-6,090,800, that requires lipid-soluble steroid prodrugs, the corticosteroid is most preferably a water-soluble corticosteroid. The term "water-sotuble" is defined herein as having a solubility at a temperature of 25°C of at least 10 g/l water or water buffered at neutral pH.

Water soluble corticosteroids which can be advantageously used in accordance with the present invention are alkali metal and ammonium salts prepared from corticosteroids, having a free hydroxyl group, and organic acids, such as (C₂ - C₁₂) aliphatic, saturated and unsaturated dicarbonic acids, and inorganic acids, such as phosphoric acid and sulphuric acid. Also acid addition salts of corticosteroids can advantageously be encapsulated in the vesicles, preferably liposomes, more preferably long-circulating PEG-liposomes. If more than one group in the corticosteroid molecule is available for salt formation, mono- as well as di-salts may be useful. As alkaline metal salts the potassium and sodium salts are preferred. Also other, positively or negatively charged, derivatives of corticosteroids can be used. Specific examples of water soluble corticosteroids are betamethasone sodium phosphate, desonide sodium phosphate, dexamethasone sodium phosphate, hydrocortisone sodium phosphate, hydrocortisone sodium succinate, methylprednisolone disodium phosphate, methylprednisolone sodium succinate, prednisolone sodium phosphate, prednisolone sodium succinate, prednisolamate hydrochloride, prednisone disodium phosphate, prednisone sodium succinate, triamcinolone acetonide disodium phosphate and triamcinolone acetonide disodium phosphate. Beside water-soluble corticosteroids also lipophilic corticosteroid derivatives prepared from corticosteroids having one or more free hydroxyl groups and lipophilic alihatic or aromatic carbon acids can be advantageously used. Corticosteroids esterified with one or two alkyl carbon acids such as palmityl and stearyl acid, containing more than 10 C-atoms are preferred.

Suitable corticosteroids include for example alclomethasone dipropionate, amcinonide, beclomethasone monopropionate, betamethasone 17-valerate, ciclomethasone, clobetasol propionate, clobetasone butyrate, deprodone propionate, desonide, desoxymethasone, dexamethasone acetate, diflucortolone valerate, diflurasone diacetate, diflucortolone, difluprednate, flumetasone pivalate, flunisolide, fluocinolone acetonide acetate, fluocinonide, fluocortolone pivalate, fluormetholone acetate, fluprednidene acetate, halcinonide, halometasone, hydrocortisone acetate, medrysone, methylprednisolone acetate, mometasone furoate, parametasone acetate, prednicarbate, prednisolone acetate, prednylidene, rimexolone, tixocortol pivalate and triamcinolone hexacetonide.

Of these corticosteroids, prednisolone disodium phosphate, prednisolone sodium succinate, methylprednisolone disodium phosphate, methylprednisolone sodium succinate, dexamethasone disodium phosphate and betamethasone disodium phosphate are preferred.

Steroids, devoid of glucocorticoid or mineralocorticoid action, termed angiostatic steroids have been shown to inhibit tumor growth with less glucocorticoid/mineralocorticoid related side-effects. Very good results have been achieved with such angiostatic corticosteroids, preferably steroids having a C20 ketone but lacking a C3 ketone, such as tetrahydrocortisone, tetrahydrocortisol and tetrahydro S or a functional analogue thereof.

Topical corticosteroids which undergo fast, efficient clearance as soon as these drugs become available in the general circulation, are of special interest. Examples thereof are budesonide, flunisolide and fluticasone proprionate, rimexolone, butixocort and beclomethason and its derivatives. By preparing a water-soluble form or a lipophilic derivative of the above-mentioned topical steroids and encapsulating these into microvesicles, preferably PEG liposomes, in accordance with the present invention, it is now possible to systemically administer such corticosteroids in order to come to tumor-site-specific drug delivery. Hereby adverse effects associated with systemic treatment and overcoming problems, which are inherent to the corticosteroid, such as a fast clearance, are avoided. In this respect, budesonide disodiumphosphate has appeared to be a salt of great interest.

As said, the microvesicles used in accordance with the present invention may be prepared according to methods used in the preparation of conventional liposomes and PEG-liposomes, as disclosed in *e.g.* EP-A-0 662 820 or WO 02/45688. Passive loading of the active ingredients into the liposomes by dissolving the corticosteroids in the aqueous phase is sufficient in order to reach an encapsulation as high as possible, but other methods can also be used. The lipid components used in forming the liposomes may be selected from a variety of vesicle-forming lipids, such as phospholipids, sphingolipids and sterols. Substitution (complete or partial) of these basic components by e.g. sphingomyelines and ergosterol appeared to be possible. For effective encapsulation of the, preferably water-soluble, corticosteroids in the microvesicles, thereby avoiding leakage of the drug from the microvesicles, especially phospholipid components having saturated, rigidifying acyl chains have appeared to be useful. The beneficial effects observed after one single injection of the water soluble corticosteroid containing PEG liposomes according to the invention are very favourable.

In addition, a composition used in accordance with the present invention may comprise one or more additional components

In this respect, the present invention also relates to the use of a composition according to the invention, which composition additionally comprises a heparin (derivative). Preferably, the heparin derivative does not affect the blood clotting.

In another preferred embodiment, the composition to be used in the present invention additionally comprises a component facilitating the delivery of the corticosteroid to the tumor, preferably a heparin or a heparin fragment. These components facilitating the delivery of the corticosteroids are described in more detail in US-A-5,762,918, which document is for the description of these components incorporated herein by reference.

Compositions to be used in accordance with the present invention may also suitably contain or comprise at least one compound selected from the group consisting of cytostatic agents and cytotoxic agents, preferably at least one compound selected from the group consisting of doxorubicin and taxol.

Moreover, suitable use can be made of compositions comprising at least one component selected from the group consisting of immunomodulators and immunosuppressants. Examples of such components are methotrexate, cyclophosphamide, cyclosporin, muramyl peptides, cytokines and penicillamine.

In an additional aspect, the present invention also relates to novel pharmaceutical compositions. For instance, the invention relates to a pharmaceutical composition comprising a long-circulating microvesicle, a corticosteroid contained therein and at least one compound selected from the group consisting of heparin and heparin derivatives.

Further, the present invention encompasses pharmaceutical compositions comprising a long-circulating microvesicle and a corticosteroid contained therein, wherein the corticosteroid is tetrahydrocorticosterone.

In yet another aspect, the present invention is directed to pharmaceutical compositions comprising a long-circulating microvesicle, a corticosteroid contained therein and at least one cytostatic and/or cytotoxic agent (other than a corticosteroid) preferably selected from the group consisting of anthracyclins (derivatives), topoisomerase I inhibitors and vinca-alkaloids.

Without wishing to be bound by any theory, it is hypothesized that apoptosis in the tumor core effectuated by the microvesical corticosteroids gives rise to a decreased efflux of the cytostatic agents out of the tumor. In addition, perhaps the above-mentioned encapsulation by connective tissue stimulated by the microvesical corticosteroids may also play a role.

Preferably, the pharmaceutical compositions of the invention comprise a long-circulating microvesicle in the form of a liposome comprising a non-charged vesicle-forming lipid, 0-20 mole percent of an amphipathic vesicle-forming lipid derivatised with polyethyleneglycol and 0-10 mole percent of a negatively charged vesicle-forming lipid, which liposomes have a selected mean particle diameter in the size range between about 40 - 200 nm.

The present invention will now further be illustrated by the following, non-limiting examples and the drawings, wherein:
Figure 1: Tissue distribution of ¹¹¹In-labeled liposomes at 24 h after intravenous administration in B16-tumor bearing C57BI/6 mice or C26-tumor bearing Balb/c mice. Tumors weighed approximately 1 g. Mean ± S.D, n=5 animals/experimental group;
Fig 2: Effect of dose of free or liposomal PLP on tumor growth in B16 or C26 bearing mice. Mice received a single injection with the indicated dose and formulation of PLP on the day tumors became palpable. Tumor volume 1 week later is reported. Mean ± S.D. N=5 mice/experimental group;
Fig 3: Effect of tumor size on anti-tumor effects of free or liposomal PLP in B16 or C26 bearing mice. Mice received either single or multiple injections with 20 mg/kg PLP of the indicated formulations. Arrows indicate treatment. Mean ± S.D is reported. N=5 mice/experimental group; and
Fig 4: Microscopic images of tumor tissue 1 week after treatment with 20 mg/kg liposomal PLP. Mice received a single injection of liposomal PLP at day 7 after tumor cell inoculation.

A: Arrows indicate occurrence of massive apoptosis in the tumor core.
   Asterisks indicate blood vessels;
B: Magnification of A. Arrow indicates blood vessel obstruction.

### Examples

### Materials and Methods

### Liposome preparation

Long-circulating liposomes were prepared by dissolving dipalmitoylphosphatidylcholine (DPPC) (Lipoid GmbH, Ludwigshafen, Germany), cholesterol (Chol) (Sigma, St. Louis, USA), and poly (ethylene) glycol 2000-distearoylphosphatidylethanolamine (PEG-DSPE) (Lipoid GmbH) in a molar ratio of 1.85:1.0:0.15, respectively, in chloroform:methanol (2:1 vol:vol) in a round-bottom flask. Typically batch sizes of 1000-2000 µmol total lipd were used. For the short-circulating liposome formulation, PEG-DSPE was replaced by a corresponding amount of egg phosphatidylglycerol (EPG) (Lipoid GmbH). A lipid film was made under reduced pressure on a rotary evaporator and dried under a stream of nitrogen. Liposomes were formed by addition of an aqueous solution of 100 mg/ml prednisolone disodium phopshate (PLP) (BUFA B.V. Uitgeest, The Netherlands) The water-soluble phosphate derivative of prednisolone was used to ensure stable encapsulation in the liposomes. In case of labeling of the liposomes with ¹¹¹In-oxine (Mallinckrodt Medical, Petten, The Netherlands), liposomes were formed by addition of 5 mM DTPA/10 mM Hepes/135 mM NaCl-buffer pH 7 to the lipid film, according to a procedure described by Boerman et al. in J. Nucl. Med. 36(9) (1995), 1639-44. Liposome size was reduced by multiple extrusion steps through polycarbonate membranes (Nuclepore, Pleasanton, USA) with a final pore size of 50 nm. The pore was 400 mm in the preparation of the short-circulating liposomes. Unencapsulated material was removed by dialysis with repeated change of buffer against 10 mM Hepes/135 mM NaCl-buffer pH 7 at 4°C.

The mean particle size of the long-circulating liposomes was determined by dynamic light scattering to be 0.1 µm with a polydispersity value of ~ 0.1, whereas the short-circulating liposomes had a particle size of 0.5 µm and a polydispersity of ~ 0.2. The polydispersity value varies between 0 and 1. A value of 1 indicates large variations in particle size, whereas a value of 0 indicates a complete monodisperse system. Thus, the present preparations showed limited variation in particle size. The amount of lipid in the liposome dispersion was determined by colorimetric phosphate determination according to Rouser (Lipids 5 (1970), 494-496).

The concentration of PLP in the liposomes was determined by HPLC. PLP was extracted with ethylacetate and concentrated under a stream of nitrogen. The samples were diluted in an appropriate volume of ethanol:water (1:1 mol:mol) and 100 µl sample or standard was injected on an RP-HPLC system equipped with a 100 RP-18 column (5 µm, 125 x 4 mm). Acetonitrile:water (75%:25% vol:vol) pH 2 was calculated using Millenium software (Waters Associates Inc.). Liposomes contained 25-35 µg PLP/µmol lipid.

### Cells

B16 murine melanoma and C26 murine colon carcinoma cells were cultured at 37°C in a 5% CO₂-containing humidified atmosphere in DMEM medium (Gibco, Breda, The Netherlands) containing 10% (v/v) fetal calf serum supplemented with 2mM L-glutamine, 100 IU/ml penicillin, 100 µg/ml streptomycin and 0.25 µg/ml amphotericin B (Gibco).

### Animals

Male Balb/c and C57B1/6 mice (6-8 weeks of age) were obtained from Charles River, kept in standard housing with standard rodent chow and water available *ad libitum,* and a 12 h light/dark cycle. Experiments were performed according to national regulations and approved by the local animal experiments ethical committee.

Tissue distribution of ¹¹¹In-labeled PEG-liposomes in tumor bearing mice

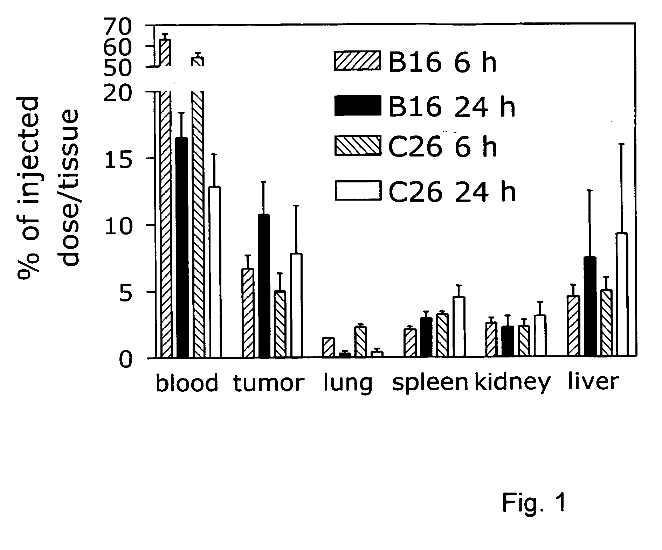

1 x 10⁵ B16 or C26 cells were inoculated subcutaneously in the flank of C57BI/6 of Balb/c mice, respectively. At a tumor volume of approximately 1 cm³, mice were injected *i*.*v*. with 25 µmol lipid/kg (corresponding to 30 x 10⁶ cpm/mouse) of ¹¹¹In-labeled liposomes. At 6 h and 24 h after injection animals were sacrificed, a blood sample was taken and tumor, lung, liver, spleen and kidneys were dissected, weighed and radioactivity was counted. See in this light Figure 1 for the results.

### Tumor growth inhibition

### Effect of dose

Mice received a single intravenous injection of an indicated dose of free PLP or liposomal PLP at the time when the tumor became palpable. At 7 days after treatment, tumor size was measured and tumor volume calculated according to the equation V = 0.52 x a² x b, wherein a is the smallest and b is the largest superficial diameter.

### Effect of tumor size

Free PLP or liposomal PLP were *i.v.* administered at a dose of 20 mg/kg at day 1, 7 and 14 or by single injection at day 7 or day 14 after tumor cell inoculation. As a reference, B16F10 tumors became palpable around 7 days and C26 tumors around 11 days after tumor cell inoculation. Tumor size was measured regularly, and tumor volume was calculated as described above.

### Statistical analysis

Data were analysed by one-way ANOVA with Dunnett's post test using GraphPad InStat version 3.05 for Windows, GraphPad Software (San Diego, USA). Data were logarithmically transformed to correct for significant differences between SD of groups, when appropriate according to Bartlett's test. Spearman rank correlation coefficient was calculated to identify dose-response.

### Results

### Tissue distribution of long-circulating liposomes

The tissue distribution of long-circulating liposomes after i.v. injection in C26 or B16-tumor bearing mice is shown in figure 1. 15% of the injected dose of radioactivily labeled liposomes was still present in the circulation in both mouse models at 24 h after injection.

Approximately 7-10% of the injected dose could be recovered from tumor tissue in both the C26 and B16F10 model. Approximately the same amount was present in the livers of both experimental groups. Relatively low amounts of liposomes were recovered form spleen, kidney and lung in the two mouse strains.

### Effect of dose of free of liposomal PLP on tumor growth

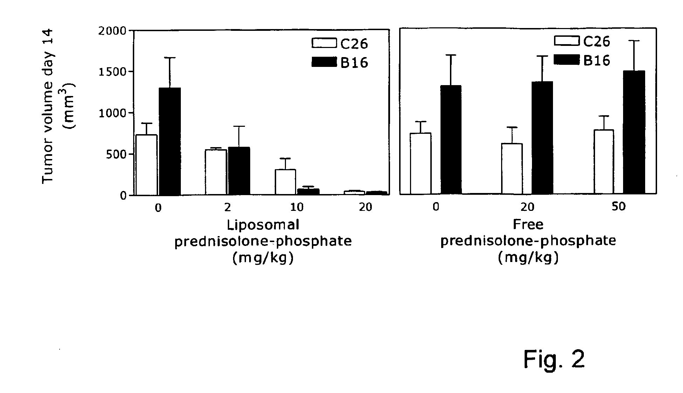

To compare the effect of different doses of free PLP or liposomal PLP on tumor growth, B16 or C26-tumor bearing mice received a single injection of either formulation at the moment that the tumor became palpable. At 1 week after injection tumor volume was smaller with increasing dose of liposomal PLP in both mouse models (B16: Spearman correlation coefficient r=-0.92 (p<0.001); C26 Spearman correlation coefficient r=-0.82 (p<0.01)). 20 mg/kg PLP was the maximum dose that could be administered for the liposomal formulation in view of injection volume.
Treatment of B16 or C26 tumor bearing mice with 20 mg/kg or 50 mg/kg free PLP did not result in significantly different tumor volumes compared to buffer treated control animals. See in this light, Figure 2 showing the results.

### Effect of tumor size

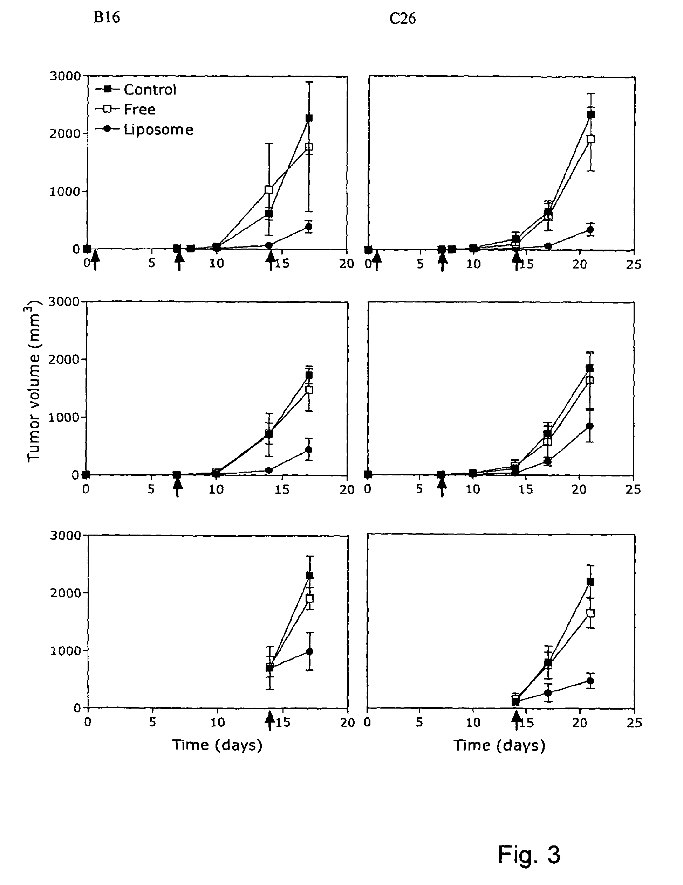

To determine the effect of time of injection of free or liposomal PLP on tumor growth inhibition, the formulations were injected at a dose of 20 mg/kg at day 1,7 and 14 or single injection at day 7 or day 14. See in this light, Figure 3, showing the results.

### B16-model

Neither liposomal PLP nor free PLP inhibited tumor growth in B16-tumor bearing mice between day 1 and day 7. Tumors were just palpable at this time-point in all groups.

Between day 7 and day 14, after a second injection at day 7, liposomal PLP resulted in 92% tumor growth inhibition as compared to controls (p<0.05), whereas free PLP did not reduce tumor volume. On day 14, mice received a third injection. At day 17, some of the mice in the free PLP and control group had to be culled because of large tumor sizes (>2 cm³), whereas average tumor volume in the liposomal PLP group was approximately 79% smaller (p<0.01).

After single injection of liposomal of free PLP at day 7, a significantly smaller tumor volume was only seen after treatment with liposomal PLP with average inhibition of tumor growth of 89% at day 14 and 67% at day 17 as compared to controls (p<0.05, both time-points). Single injection at day 14 showed a similar image, at day 17 liposomal PLP treated tumors were 58% smaller than controls (p<0.05).

### C26-model

Neither liposomal PLP nor free PLP inhibited tumor growth in C26-tumor bearing mice between day 1 and day 7. Mice received a second injection on day 7. Tumors became palpable around day 10. Both at day 14 and day 21, average tumor volume in liposomal PLP-treated animals was 89% smaller than that of controls, but it was only significantly smaller at day 21 (p<0.01). Free PLP did not inhibit tumor growth. After single injection of liposomal of free PLP at day 7, tumor volume was not significantly smaller with either treatment compared to controls. Although average tumor volume was 66% at day 14 and 67% at day 21 smaller for liposomal PLP treated mice as compared to controls. Single injection at day 14 resulted in a 78% smaller tumor volume at day 21 for liposomal PLP treated animals (p<0.05).

### Effect of liposomal circulation time

To determine whether the liposome formulation is important in therapeutic efficacy we tested a short-circulating and long-circulating liposome formulation of PLP. Both were injected at day 14 after tumor cell inoculation in C26 tumor bearing mice. It appeared that the tumor inhibition of short-circulating liposomes was not as pronounced and lasted shorter than that of long-circulating and was not significantly different form saline treated animals.

### Analysis of amount of PLP or PL in tissues

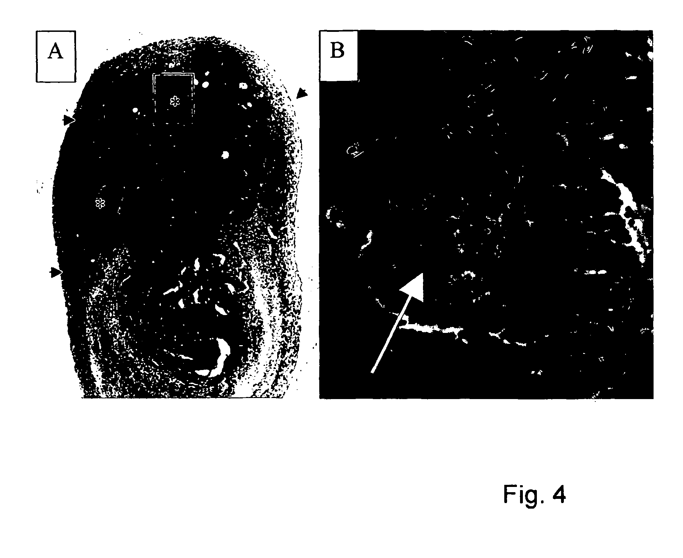

PLP and prednisolone (PL) concentrations at 24 h after injection of liposomal PLP in liver, spleen and tumor tissue were determined by HPLC analysis. Figure 4 shows that the highest amount of PLP (± 5 µg) was present in the tumor, which was a similar amount as present in the form of PL. Levels of PLP or PL in the spleen were relatively low. In liver tissue, hardly any PLP could be detected, but a high amount of PL was present. As PLP added to control liver tissue to prepare the standard line could also not be detected, the PL content of this tissue is likely overrated as a result of high enzymatic activity in liver homogenates. Neither PLP nor PL was detected in any of these tissues at 24 h after injection of free PLP.

Microscopical evaluation of tumor tissue treated with liposomal PLP at 1 week after a single dose of 20 mg/kg showed massive apoptosis of tumor cells in the tumor core. In addition, the presence of blood clots in larger blood vessels obstructing tumor blood flow, is shown. The results are shown in Figure 4.

## Claims

1. Use of a composition comprising a corticosteroid encapsulated in a long-circulating microvesicle for the manufacture of a medicament for the treatment of solid primary and/or secondary tumors of a non-lymphatic cancer.

2. Use according to claim 1, wherein the microvesicle is a liposome, nano-capsule or a polymeric micelle, which microvesicle has a neutral or negative charge at physiological conditions.

3. Use according to claim 1 or 2, wherein the microvesicles are liposomes comprising a non-charged vesicle-forming lipid, 0-20 mole percent of an amphipathic vesicle-forming lipid derivatised with polyethyleneglycol, 0-50 mole percent of a sterol, and 0-10 mol % of a negatively charged vesicle-forming lipid, which liposomes have a selected mean particle diameter in the size range between about 40 - 200 nm.

4. Use according to any one of claims 1-3, wherein the microvesicle has a circulation half-life of at least 6 hours.

5. Use according to any one of the preceding claims, wherein the medicament is a medicament for parental or local application.

6. Use according to any of the preceding claims, wherein the composition comprises at least one agent affecting the blood clotting cascade.

7. Use according to any one of the preceding claims, wherein the composition additionally comprises a component interacting with the corticosteroid on the tumor, preferably a heparin or a heparin fragment.

8. Use according to any one of the preceding claims, wherein the composition comprises at least one compound selected from the group consisting of cytostatic agents and cytotoxic agents, preferably comprising at least one compound selected from the group consisting of anthracyclins, doxorubicin, taxol topoisomerase I inhibitors and vinca-alkaloids.

9. Use according to any one of the preceding claims, wherein the composition comprises at least one component selected from the group consisting of immunomodulators and immunosuppressants.

10. Use according to any one of the preceding claims, wherein the corticostoroid is a water-soluble, and preferably an angiostatic corticosteroid, more preferably tetrahydrocorticosterone or a functional analogue thereof.

11. Pharmaceutical composition comprising a long-circulating microvesicle, a corticosteroid contained therein and at least one compound selected from the group consisting of heparin and heparin derivatives.

12. Pharmaceutical composition comprising a long-circulating microvesicle and a water-soluble corticosteroid contained therein, wherein the corticosteroid is tetrahydrocorticosterone.

13. Pharmaceutical composition comprising a long-circulating microvesicle, a corticosteroid contained therein and at least one cytostatic and/or cytotoxic agent (other than a corticosteroid) selected from the group consisting of doxorubicin and taxol.

14. Pharmaceutical composition according to any one of the claims 11-13, wherein the long-circulating microvesicle is a liposome comprising a non-charged vesicle-forming lipid, 0-20 mole percent of a polymer-lipid conjugate and 0-10 mole percent of a negatively charged vesicle-forming lipid, which liposomes have a selected mean particle diameter in the size range between about 40 - 200 nm.

## Patentansprüche

1. Verwendung einer Zusammensetzung, die ein Corticosteroid verkapselt in einem lange zirkulierenden Mikrovesikel umfasst, zur Herstellung eines Medikaments zur Behandlung von festen primären und/oder sekundären Tumoren eines nichtlymphatischen Krebses.

2. Verwendung nach Anspruch 1, bei der das Mikrovesikel ein Liposom, eine Nanokapsel oder eine polymere Mizelle ist, wobei das Mikrovesikel unter physiologischen Bedingungen eine neutrale oder negative Ladung aufweist.

3. Verwendung nach Anspruch 1 oder 2, bei der die Mikrovesikel Liposomen sind, die ungeladenes, Vesikel-bildendes Lipid, 0 bis 20 Mol.% amphiphatisches Vesikel-bildendes Lipid, das mit Polyethylenglykol derivatisiert ist, 0 bis 50 Mol.% Sterol und 0 bis 10 Mol.% negativ geladenes Vesikel-bildendes Lipid umfasst, wobei die Liposomen einen ausgewählten mittleren Teilchendurchmesser im Größenbereich von 40 bis 200 nm aufweisen.

4. Verwendung nach einem der Ansprüche 1 bis 3, bei der die Mikrovesikel eine Zirkulationshalbwertslebensdauer von mindestens 6 Stunden aufweisen.

5. Verwendung nach einem der vorhergehenden Ansprüche, bei der das Medikament ein Medikament zur parenteralen oder lokalen Applikation ist.

6. Verwendung nach einem der vorhergehenden Ansprüche, bei der die Zusammensetzung mindestens ein Mittel umfasst, das die Blutgerinnungskaskade beeinflusst.

7. Verwendung nach einem der vorhergehenden Ansprüche, bei der die Zusammensetzung außerdem eine Komponente umfasst,: die mit dem Corticosteroid an dem Tumor in Wechselwirkung tritt, vorzugsweise ein Heparin oder ein Heparinfragment.

8. Verwendung nach einem der vorhergehenden Ansprüche, bei der die Zusammensetzung mindestens eine Verbindung ausgewählt aus der Gruppe bestehend aus zytostatischen Mitteln und zytotoxischen Mitteln umfasst, die vorzugsweise mindestens eine Verbindung ausgewählt aus der Gruppe bestehend aus Anthracyclinen, Doxorubicin, Taxol-Topoisomerase I-Inhibitoren und Vinca-Alkaloiden umfassen.

9. Verwendung nach einem der vorhergehenden Ansprüche, bei der die Zusammensetzung mindestens eine Komponente ausgewählt aus der Gruppe bestehend aus Immunmodulatoren und Immunsuppressiva umfasst.

10. Verwendung nach einem der vorhergehenden Ansprüche, bei der das Corticosteroid ein wasserlösliches und vorzugsweise ein angiostatisches Corticosteroid ist, bevorzugter Tetrahydrocorticosteron oder ein funktionelles Analoges desselben.

11. Pharmazeutische Zusammensetzung, die ein lange zirkulierendes Mikrovesikel, ein darin enthaltenes Corticosteroid und mindestens eine Verbindung ausgewählt aus der Gruppe bestehend aus Heparin und Heparinderivaten umfasst.

12. Pharmazeutische Zusammensetzung, die ein lange zirkulierendes Mikrovesikel und ein darin enthaltendes wasserlösliches Corticosteroid umfasst, wobei das Corticosteroid Tetrahydrocorticosteron ist.

13. Pharmazeutische Zusammensetzung, die ein lange zirkulierendes Mikrovesikel, ein darin enthaltenes Corticosteroid und mindestens ein zytostatisches und/oder zytotoxisches Mittel (verschieden von einem Corticosteroid) ausgewählt aus der Gruppe bestehend aus Doxorubicin und Taxol umfasst.

14. Pharmazeutische Zusammensetzung nach einem der Ansprüche 11 bis 13, bei der das lange zirkulierende Mikrovesikel ein Liposom ist, das ungeladenes, Vesikel-bildendes Lipid, 0 bis 20 Mol.% Polymer-Lipid-Konjugat und 0 bis 10 Mol.% negativ geladenes, Vesikel-bildenden Lipid umfasst, wobei die Liposomen einen ausgewählten mittleren Teilchendurchmesser im Größenbereich von 40 bis 200 nm aufweisen.

## Revendications

1. Utilisation d'une composition comprenant un corticostéroïde encapsulé dans une microvésicule à circulation prolongée pour la fabrication d'un médicament pour le traitement des tumeurs solides primaires et/ou secondaires d'un cancer non lymphatique.

2. Utilisation selon la revendication 1, dans laquelle la microvésicule est un liposome, une nanocapsule ou une micelle polymère, ladite microvésicule ayant une charge neutre ou négative dans des conditions physiologiques.

3. Utilisation selon la revendication 1 ou 2, dans laquelle les microvésicules sont des liposomes comprenant un lipide formant une vésicule non chargé, de 0 à 20 pour cent en mole d'un lipide formant une vésicule bipolaire dérivé avec du polyéthylèneglycol, de 0 à 50 pour cent en mole d'un stérol, et de 0 à 10 pour cent en mole d'un lipide formant une vésicule chargé négativement, lesdits liposomes ayant un diamètre particulaire moyen choisi dans la gamme comprise entre environ 40 et 200 nm.

4. Utilisation selon l'une quelconque des revendications 1 à 3, dans laquelle la microvésicule a une demi-vie de circulation d'au moins 6 heures.

5. Utilisation selon l'une quelconque des revendications précédentes, dans laquelle le médicament est un médicament destiné à être appliqué par voie parentérale ou par voie locale.

6. Utilisation selon l'une quelconque des revendications précédentes, dans laquelle la composition comprend au moins un agent affectant les réactions en cascade de la coagulation sanguine.

7. Utilisation selon l'une quelconque des revendications précédentes, dans laquelle la composition comprend en outre un composant interagissant avec le corticostéroïde sur la tumeur, de préférence une héparine ou un fragment d'héparine.

8. Utilisation selon l'une quelconque des revendications précédentes, dans laquelle la composition comprend au moins un composant choisi dans le groupe constitué par les agents cytostatiques et les agents cytotoxiques, comprenant de préférence au moins un composé choisi dans le groupe constitué par les anthracyclines, la doxorubicine, le taxol, les inhibiteurs de topoisomérase I et les vinca-alcaloïdes.

9. Utilisation selon l'une quelconque des revendications précédentes, dans laquelle la composition comprend au moins un composant choisi dans le groupe constitué par les immunomodulateurs et les immunosuppresseurs.

10. Utilisation selon l'une quelconque des revendications précédentes, dans laquelle le corticostéroïde est un corticostéroïde hydrosoluble et de préférence angiostatique, de préférence la tétrahydrocorticostérone ou un analogue fonctionnel.

11. Composition pharmaceutique comprenant une microvésicule à circulation prolongée, un corticostéroïde contenu dans cette vésicule et au moins un composé choisi dans le groupe constitué par l'héparine et les dérivés de l'héparine.

12. Composition pharmaceutique comprenant une microvésicule à circulation prolongée et un corticostéroïde hydrosoluble contenu dans cette vésicule, dans laquelle le corticostéroïde est la tétrahydrocorticostérone.

13. Composition pharmaceutique comprenant une microvésicule à circulation prolongée, un corticostéroïde contenu dans cette vésicule et au moins un agent cytostatique et/ou cytotoxique (autre qu'un corticostéroïde) choisi dans le groupe constitué par la doxorubicine et le taxol.

14. Composition pharmaceutique selon l'une quelconque des revendications 11 à 13, dans laquelle la microvésicule à circulation prolongée est un liposome comprenant un lipide formant une vésicule non chargé, de 0 à 20 pour cent en mole d'un conjugué polymère-lipide et de 0 à 10 pour cent en mole d'un lipide formant une vésicule chargé négativement, lesdits liposomes ayant un diamètre particulaire moyen choisi dans la gamme comprise entre environ 40 et 200 nm.
